# EUROPEAN PATENT APPLICATION

(11) **EP 2 053 399 A1**
(43) Date of publication of application: **29.04.2009**
(21) Application number: 07118613.4
(22) Date of filing: 16.10.2007
(51) Int. Cl.: G01N 33/542, G01N 33/543, G01N 33/50

(54) **A chemotaxis essay based on oscillating gradients of chemotactic agents**

(71) Applicant: Koninklijke Philips Electronics N.V., 5621 BA Eindhoven (NL)
(72) Inventor: The designation of the inventor has not yet been filed
(74) Representative: Golla-Franz, Anke Lucia

(57) **Abstract**

The present invention is concerned with the method of detecting movement of cells. The present invention is further concerned with providing a device for detecting movement of cells.

## Description

### SUBJECT OF THE INVENTION

The present invention is concerned with the method of detecting movement of cells. The present invention is further concerned with providing a device for detecting movement of cells.

### BACKGROUND OF THE INVENTION

Cell migration is a key physiological process among prokaryotes as well as eukaryotes.

Movement of cells through e.g. the human body is a complex cellular process that is mediated by a plethora of signals including ligands and receptors such as e.g. the integrin receptor families. Growth factors are another type of molecules, which are frequently involved in cellular movement at least in case of mammalians. Further, for mammalians and particularly humans it has been shown that impaired cell migration can be involved in pathological processes.

For example, coronary artery diseases such as atherosclerosis are characterized by the movement of monocytes, which is induced by growth factors such as e.g. vascular endothelial growth factor (VEGF). The monocytes then travel until they contact endothelial cells at the site where e.g. an inflammation has occurred. Contacting and adherence of the monocytes is mediated e.g. by vascular cell adhesion matrix protein-1 (VCAM-1).

In atherosclerosis, monocytes adhere to endothelial cells. then invade into the layers below the endothelial cells, turn into macrophages and take up cholesterol to ultimately result in foam cells, which are one major constituents of atherosclerotic plaques.

In view of the fundamental physiological importance of cell movement, different assays have been developed in order to investigate cell migration in an in-vitro setting, e.g. outside the human or animal body.

One of the most common two-dimensional chemotaxis migration assays makes use of the so-called Boyden chamber assay. Another typical migration assay is based on the so-called Dunn chamber.

All of these assays have certain advantages and disadvantages and have in common that it usually takes a rather elongated time period in order to assess the chemotactic behavior of certain cell type. An overview on various chemotaxis assays can be found in Entschladen et al. (Exp. Cell. Res., (2005), 307 (2): 418-426).

Nevertheless there is a continuing need for further chemotaxis assays that allow efficient and straightforward analysis of a cell's capacity to migrate and to react towards a certain migratory stimuli.

### OBJECT AND SUMMARY OF THE INVENTION

It is one objective of the present invention to provide a method, which allows monitoring the migratory behavior of cells in a convenient and efficient setup.

It is a further objective of the present invention to provide devices that allow performing such methods.

These objectives as well as others, which will become apparent from the ensuing description, are attained by the subject matter of the independent claims. Some of the preferred embodiments of the present invention are defined by the dependent claims.

The present invention in one embodiment relates to a method of detecting movement of at least one cell comprising at least the steps of:
a) Providing at least one support structure;
b) Providing a sample with at least one cell;
c) Allowing said at least one cell to reversibly adhere to at least a part of said support structure
d) Establishing a gradient of a chemotactic agent, which is capable of inducing chemotaxis of said at least one cell along of at least a part of said support structure;
e) Monitoring movement and/or adherence of said at least one cell to or from said gradient;
f) Changing the direction of said gradient;
g) Monitoring movement of said at least one cell to or from said gradient.

In one of the embodiments of the present invention steps d) to g) are repeated over time. This allows generating oscillating gradient and monitor movement in response to such oscillating gradients. The advantages of monitoring movement of cells with respect to such oscillating gradients will be described hereinafter.

The chemotactic agent may be a chemoattractant or a chemorepellent.
Specific examples of such agents are provided hereinafter.

In a further embodiment of the present invention the support structure may comprise at least one probe molecule that allows cells to be monitored to adhere to the support structure. These probe molecules may be selected from the group comprising small molecules, peptides, glycopeptides, proteins, glycoproteins, receptors, receptor ligands, antibodies, growth factors, hormones, cytokines, chemokines, cell adhesion molecules, integrins, vitamins, nucleic acids and derivatives thereof, lipids and glycans.

In one embodiment of the present invention the chemotactic agent and the probe molecules may be identical.

In one aspect of the methods in accordance with the present invention monitoring movement of cells will include determination of certain parameters including trajectories, time lag of cells for responding to a change in the gradient, adhesion tendency and/or mobility of cells.

One of the preferred embodiments of methods in accordance with the invention use Total Internal Reflection Microscopy (TIRFM) monitoring movement of cells. In another embodiment the present methods use Förster Resonance Energy Transfer (FRET) for monitoring cell movement.

Yet another embodiment of the present invention relates to an apparatus comprising:
- a first inlet and a second inlet from which at least first generation of channels originates wherein said first generation comprises at least two first generation channels;
- a first common channel providing communication between each of the at least two first generation channels; and
- at least a second generation of channels comprising at least three second generation channels, each second generation channel having a first end and a second end, the first end of each being in communication with the first common channel and the second end of each being in communication with a second common channel,
- a third inlet; and
- an outlet;
wherein the apparatus is adapted to allow formation of a liquid gradient of a component by connecting the first inlet at least to one fluid comprising a component at a first concentration and by connecting the second inlet at least to one fluid comprising said component at a second concentration wherein the first and second concentration of said component differ; and
wherein said first inlet and said second inlet are adapted to allow for reversal of the orientation of the gradient.

In a preferred embodiment the first and second inlet may be three way valves.

The present invention also relates to the use of such an apparatus for detecting cell movement.

The present invention further relates to the use of the movement characteristics determined for cells in the context of diagnosing diseases.

### FIGURE LEGENDS

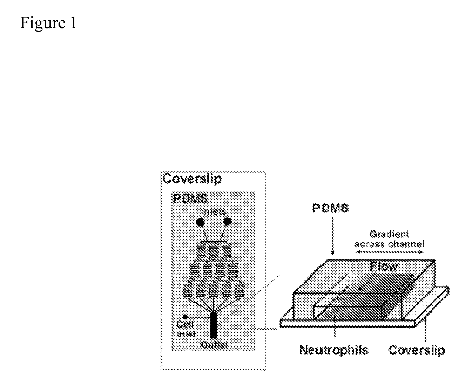

- Fig. 1: depicts schematically a device for creating a gradient of a chemotactic signal. a) Schematic drawing of the device that is used for creating a gradient. b) Schematic three-dimensional drawing that indicates the direction of the gradient formation as well as the flow device.
- Fig. 2: schematically depicts a device in accordance with the invention (1) denotes a first inlet, (2) denotes a second inlet, (3) denotes the first generation of channels, (4) denotes the first common channel, (5) denotes the second generation of channels, (6) denotes the second generation common channel, (7) denotes the third generation of channels, (8) denotes a third inlet, (9) denotes an outlet. Further, the direction of the gradient for the chemoattractant (CA) is depicted. In this specific example inlets (1) and (2) are three way valves.
- Fig. 3: depicts the same schematic device as in Fig. 2 except the three way valves for inlet (1) and (2) are in different positions so that the direction of gradient for the chemotactic agent (CA) is reversed.

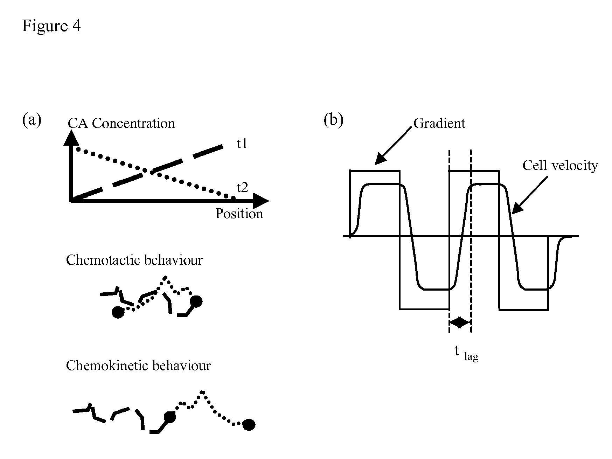

- Fig. 4: schematically depicts different applications of oscillating gradients. a) depicts how oscillating gradients are used to distinguish between chemotactic and chemokinetic migratory behavior, b) depicts the time lag of migratory behavior of cells responding to an oscillatory gradient.
- Fig. 5: schematically depicts how the correlation between cell mobility and cell adhesion is measured.
- Fig. 6: schematically depicts the use of TIRFM for monitoring cell movement.

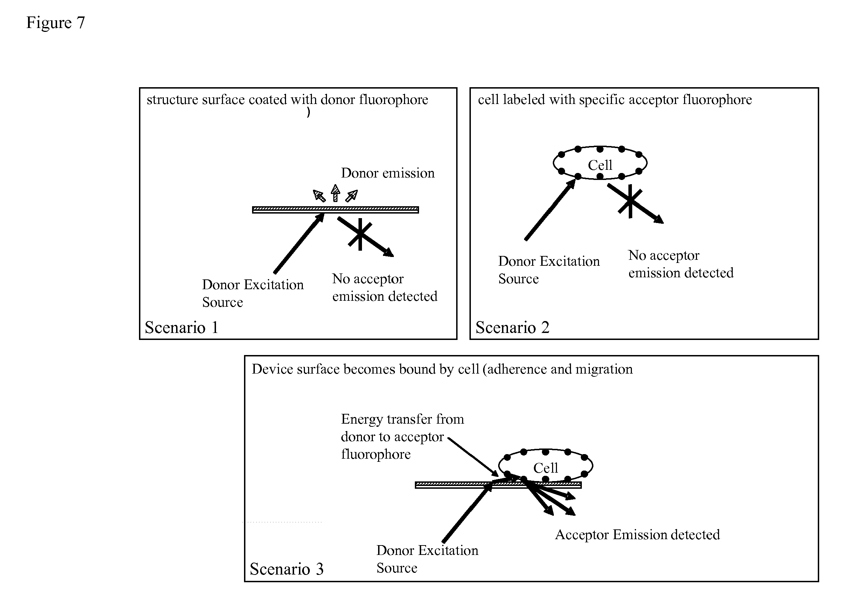

- Fig. 7: schematically depicts the use of a FRET for monitoring cell movement.

### DETAILED DESCRIPTION OF THE INVENTION

The present invention is based on the finding that gradients of chemotactic agents, which change their direction, can be used to accurately determine the migratory behavior of different cell types. In particular, inventors of the present invention have found that oscillating gradients can be used to differentiate between chemotactic and chemokinetic migratory behavior. The inventors of the present invention have further found that Total Internal Reflection Microscopy (TIRFM) or Förster Resonance Energy Transfer (FRET) may be particularly suited to mechanistic aspect of migratory behavior.

Before the invention will be described in detail with respect to some of its preferred embodiments, the following general definitions are provided.

As used in this specification and in the appended claims, the singular forms of "a" and "an" also include the respective plurals unless the context clearly dictates otherwise. Thus, the term "a probe molecule" can include more than one probe molecule, namely two, three, four, five etc. probe molecule.

The terms "about" or "approximately" in the context of the present invention denotes an interval of accuracy that the person skilled in the art will understand to still ensure the technical effect of the feature in question. The term typically indicates deviation from the indicated numerical value of +/- 10%, and preferably +/- 5%.

It is to be understood that the term "comprising" is not limiting. For the purposes of the present invention, the term "consisting of" is considered to be a preferred embodiment of the term "comprising of". If hereinafter a group is defined to comprise at least a certain number of embodiments, this is also meant to disclose a group, which preferably consists only of these embodiments.

Further definitions of terms will be given in the context of which the terms are used.

As mentioned above, the present invention in one embodiment relates to a method of detecting movement of at least one cell comprising at least the steps of:
a) Providing at least one support structure;
b) Providing a sample with at least one cell;
c) Allowing said at least one cell to reversibly adhere to at least a part of said support structure
d) Establishing a gradient of a chemotactic agent, which is capable of inducing chemotaxis of said at least one cell along of at least a part of said support structure;
e) Monitoring movement and/or adherence of said at least one cell to or from said gradient;
f) Changing the direction of said gradient;
g) Monitoring movement of said at least one cell to or from said gradient.

In one embodiment of the present invention, all of the above-mentioned steps may be performed outside the human or animal body.

The above-mentioned at least one support structure may be any type of support structure that can be used to allow a cell to adhere to.

Thus, the support structure may e.g. be made from glass or plastic materials such as plastics coated with e.g. poly lysine. Typically a microscope cover slide may be a suitable support structure.

In the context of the present invention the term "cell" denotes cells and cell types, the migratory behavior of which is to be examined. Thus, in principle the term may relate to any type of cell that can be isolated from a biological sample such as bodily fluids, including blood, plasma, urine etc. However, samples may also comprise environmental samples taken from soils, lakes, rivers, plants etc.

As will be set out hereinafter, the methods in accordance with the invention may also be used in the context of diagnosing a certain type of disease. Thus, the present invention in a preferred embodiment focuses on cells that are known to be capable of migrating and for which it is known that they are involved in certain types of diseases. Thus, the cells may e.g. be bacteria.

In case of mammalian and particularly human cells such cells may thus be selected from the group comprising embryonic, fetal or adult stem cells, progenitor cells, blood cells, fibroblast, tumor cells and neuronal cells. Other cell types include granulocytes and endothelial cells. As regards blood cells, monocytes, macrophages, B and T lymphocytes, neutrophils, basophils and eosinophils may be preferred.

In methods in accordance with the invention, 'the cells are allowed to reversibly adhere to at least a part of the aforementioned support structure. The term "reversible adherence" in the context of the present invention means that the cell can adhere to the support structure but do not permanently stick to it and keep the capacity to move along the support structure as they encounter migratory stimuli.

In one aspect of the present invention the support structure may be modified with probe molecules. These probe molecules may display an affinity for the cells to be examined and thus allow the cells to more efficiently adhere to the support structure. Furthermore, if only certain parts of the support structure are modified with such probe molecules it may be possible to create support structures which comprise cells in distinct spots only which will make it then easier to monitor the migratory behavior of the cells.

Probe molecules may be selected from the group comprising small molecules, peptides, glycopeptides, proteins, glycoproteins, receptors, receptor ligands, antibodies, growth factors, hormones, cytokines, chemokines, cell adhesion molecules, integrins, vitamins, nucleic acids and derivatives thereof, lipids and glycans.

Growth factors and cell adhesion molecules may be preferred as probe molecules. Typical growth factors include e.g. human growth factor, human nerve growth factor, platelet derived growth factor, TGFß, EGF, VEGF, TNF-a,TNF-b, FGFs, TGF-a, Epo, IGF-I, IGF-II, IL-1, IL-2, IL-6, IL-8, INF-g and CSFs as well as integrins and CAMs including VLA-1 to VLA-6, LPAM-1, LFA-1, CR3, CR4, leukointegrin, collagen, laminin, fibronectin, VCAM-1, MAdCAM-1, E-cadherin, ICAM-1, ICAM-2, ICAM-3, C3b, C4b.

A particularly preferred growth factor will be VEGF. A particularly preferred cell adhesion molecule will be vascular endothelial cell adhesion molecule-1 (VCAM-1).

The person skilled in the art will also be aware that fragments (both synthetic peptide and proteolytically cleaved fragments) of the aforementioned molecules can be used as probe molecules as long as such fragments provide the necessary binding specificity.

The association of the at least one support structure and at least one probe molecule may be achieved by covalent or non-covalent interaction between the support structure and the probe molecule. In case of a non-covalent interaction, it is nevertheless intended that the probe structures in accordance with the present invention do not significantly release the probe molecules during the time periods over which the methods in accordance with the invention are typically performed. Thus, it is currently not envisaged that the support structures should release the probe molecules to e.g. establish a signal gradient of the probe molecule. Rather, a substantially permanent association of the probe molecule and the support structure is intended.

In one of the preferred embodiments, the probe molecule may be covalently attached to the support structure. This may be achieved by functionalizing e.g. the surface of the support structures, which may be cover slides. In the following, functionalization of carrier structures will be discussed mainly with respect to such cover slides. However, the person skilled in the art understands that similar principals will also apply to other support structures.

A person skilled in the art is familiar how to functionalize e.g. the surface of a glass cover slide with the aforementioned probe molecules.

To this end, one may e.g. coat the glass cover slide with a self-assembling polymer. Such a self-assembling polymer may e.g. be polyethylenglycol. The polymers may be further functionalised to introduce chemical functionalities that allow e.g. covalent coupling of an antibody to the surface of the glass cover slide.

If e.g. growth factors or cell adhesion molecules such as VEGF and VCAM-1 are used as a probe and should be covalently coupled to a glass cover slide, slides may be used that provide functional groups such as carboxyl groups, amine groups, hydroxyl groups, sulfhydryl groups etc. These groups may then be cross-linked either directly to the growth factors or cell adhesion molecules such as VEGF and VCAM-1 or using a linker that may be homo-or hetero-bifunctional.

Thus, glass cover slide may be activated for providing a chemical linkage to the probe molecules by e.g. coating the slides with a polymer having e.g. acyl fluoride functionalities. Other covalent attachment chemistries include but are not limited to anhydrides, epoxides, aldehydes, hydrazides, acyl azides, aryl azides, diazo compounds, benzophenones, carbodiimides, imidoesters, isothiocyanates, NHS esters, CNBr, maleimides, tosylates, tresyl chloride, maleic acid anhydrides and carbonyldiimidazole.

As has been mentioned above, a particular preferred type of probe molecule that can be used to modify a glass cover slide are molecules that activate adhesion of a specific cell type. Thus, in a particularly preferred embodiment, the present invention makes use of, which are modified to display VEGF and/or VCAM-1. The function of VEGF has been mentioned above. The cell adhesion molecule VCAM-1 is known to specifically bind leukocytes, thus allowing for separation of white blood cells from plasma, platelets and red blood cells in whole blood samples.

The person skilled in the art will be able to identify further suitable probe molecules that are known to detect e.g. specifically a certain cell type. A person skilled in the art will furthermore be capable of identifying suitable attachment chemistry for either covalently or non-covalently associating such a probe molecule with support structures.

For functionalising of glass cover slides with e.g. VEGF or VCAM-1, VEGF or VCAM-1 can be coupled to the surfaces of such slides, which have been functionalised with a reactive group such as e.g. succinimide esters. The succinimide esters will preferably react with primary amines within VEGF or VCAM-1 to form a covalent linkage.

After the cells to be examined have adhered to the support structure, which may or may not be further modified with the above-mentioned probe molecules, a gradient of a chemotactic agent is established along of at least a part of the aforementioned support structure.

In the context of the present invention the term "chemotactic agent" denotes chemoattractants as well as chemorepellents.

Chemoattractants are understood to stimulate migration of cells towards these agents while chemorepellents are understood to stimulate the opposite behavior.

In the context of the present invention the term "chemoattractant" typically denotes chemical substances as well as peptides and proteins as they are known to induce migration of cells. Such chemoattractants typically include growth factors, hormones, peptides, cytokines, chemokines, cell adhesion molecules (CAMs), integrins, drugs and vitamins.

Chemoattractants such as growth factors, hormones, cytokines, chemokines can be preferred, as they are known to attract and induce a migratory behavior in eukaryotic cells such as human or animal mammalian cells.

Typical growth factors include e.g. human growth factor, human nerve growth factor, platelet derived growth factor, TGFß, EGF, VEGF, TNF-a,TNF-b, FGFs, TGF-a, EPO, IGF-I, IGF-II, IL-1, IL-2, IL-6, IL-8and CSFs as well as integrins and CAMs including VLA-1 to VLA-6, LPAM-1, LFA-1, CR3, CR4, leukointegrin, collagen, laminin, fibronectin, VCAM-1, MAdCAM-1, E-cadherin, ICAM-1, ICAM-2, ICAM-3, C3b, C4b. Other growth factors include CCL 1-28, CXCL 1-16, XCL1, XC:2, CX3CL1, IL 1-22, INF α, β and y, M-CSF, G-CSF, GM-CSF, MIF, any recognized CD marker of hematopoetic cells (CD 1-339), PDGF, NGF, TPO, GDF-8, GDF-9, bFGF, HGF, FGF, DII4 and extracellular matrix components such as collagen, fibronectin and laminin.
The person skilled in the art is aware that the above-mentioned at least one probe molecule and the chemotactic agent may be identical even though this is not a necessity. In one preferred embodiment the probe molecule may be VCAM-1 while the gradient is established using VEGF.

After having established a gradient of chemotactic agent, movement of said at least one cell with respect to the gradient of chemotactic agent is monitored. Thus, if the chemotactic agent is a chemoattractant one will observe movement of said at least one cell towards increasing concentration of the chemotactic agent. If however the chemotactic agent is a chemorepellent one will observe cells move away from increasing concentrations of the chemotactic agent towards those areas of the gradient which comprise lower concentrations of the chemotactic agent.

In the following, some embodiments of the invention will be discussed with respect to chemotactic agents that are chemoattractants. However, the person skilled in the art will be aware that the statements made in this context apply *mutatis mutandis* also to cases where a gradient is made from a chemorepellent agent.

Once one has monitored movement of said cell with respect to the gradient, the direction of the gradient is changed and then again movement of the cells with respect to the reversed gradient is observed. A preferred embodiment may relate to methods where the direction of the gradient is reversed.

By repeatedly establishing a gradient, monitoring movement of the cell, changing the gradient's direction and again monitoring movement of the cell it is possible to generate oscillating gradients and to monitor movement of cells with respect to such oscillating gradients. The advantages of monitoring movement of cells with respect to oscillating gradients will be described hereinafter.

The person skilled in the art is aware how to establish oscillating gradients of reversing direction. For example, US 6,705,357 describes devices that can be used to establish gradients.

In addition, the present invention provides an apparatus that can be used to generate oscillating gradients.

In one embodiment the present invention thus relates to an apparatus comprising:
- a first inlet and a second inlet from which at least first generation of channels originates wherein said first generation comprises at least two first generation channels;
- a first common channel providing communication between each of the at least two first generation channels; and
- at least a second generation of channels comprising at least three second generation channels, each second generation channel having a first end and a second end, the first end of each being in communication with the first common channel and the second end of each being in communication with a second common channel,
- a third inlet; and
- an outlet;
wherein the apparatus is adapted to allow formation of a liquid gradient of a component by connecting the first inlet at least to one fluid comprising a component at a first concentration and by connecting the second inlet at least to one fluid comprising said component at a second concentration wherein the first and second concentration of said component differ; and
wherein said first inlet and said second inlet are adapted to allow for reversal of the orientation of the gradient.

In a preferred embodiment the first and second inlet will be three way valves with the first and second inlet both being connected to fluids comprising different concentrations of chemotactic agent.

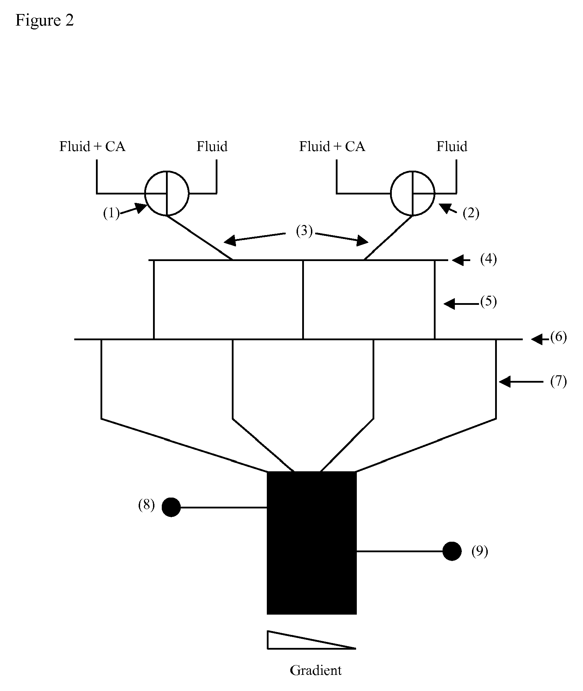

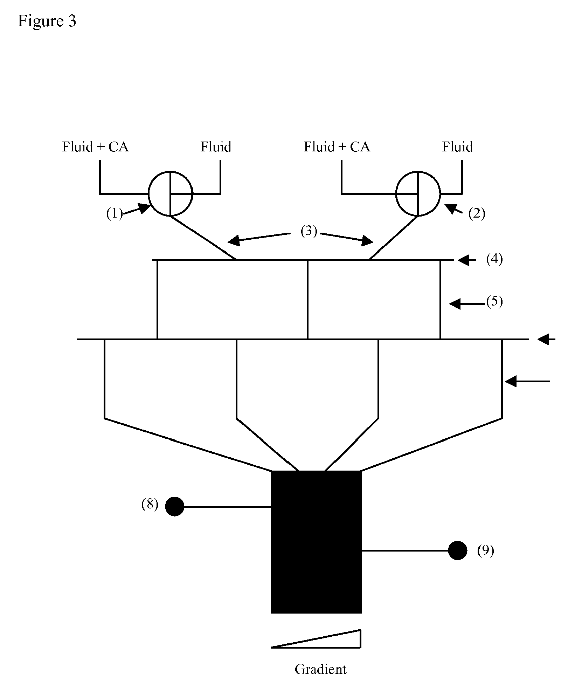

This preferred embodiment of the present invention is depicted in Figs. 2 and 3, which describe the same apparatus with different settings of the three-way valve in inlet (1) and inlet (2). In the following the apparatus will be discussed with respect to Fig. 2. Thus, the apparatus comprises a first inlet (1) and a second inlet (2). The first inlet and the second inlet each are connected to fluids comprising a chemotactic agent (CA) at different concentrations. Then a first generation of channels (3) descends from inlet (1) and (2). The ends of this first generation of channels which are opposite to the ends being connected to inlet (1) and (2) connect to a first common channel (4) providing communication between each of the at least two first generation channels. This results in different concentrations of the chemotactic agent along the first common channel.

Then, a second generation of channels (5) comprising at least three second generation channels descend from the first common channel and are connected to a second common channel (6). Again, along the second common channel (6) the concentration of the chemotactic agent differs. The second common channel may already allow and comprise an inlet for disposing cells into the second common channel and further provide means to monitor movement of said cells. Such means may e.g. a transparent housing, which enables viewing movement of cells by e.g. light microscopy.

In the specific embodiment depicted in Fig. 2, a third generation of channels (7) descends from the second common channel (6) to ultimately establish a gradient in a chamber which has a third inlet (8) that allows of disposing cells in the chamber. In the chamber there is a gradient of chemotactic agent and the chamber may comprise means to allow monitoring of the cells' movement along the gradient.

If the three way valves are changed into different positions the direction of the gradient can be reversed (see Fig. 3). The person skilled in the art is aware that these devices may comprise additional generation of channels and common channels being connected by those channels.

As mentioned above once such oscillating gradients are established, one can monitor movement of cells in response to the oscillating gradients. One advantage of monitoring cell movement with respect to oscillating gradient is that one can clearly differentiate between a chemotactic movement, i.e. a response to a chemotactic stimulus versus chemokinetic movement, i.e. the basal random migratory behavior of cells that is independent of a response to a chemotactic stimulus and also designated as "random walk".
One can, of course, use oscillating gradients to monitor actual migration, i.e. mean displacement from the original position.

However, another advantage of using oscillating gradients is, that one does not have to measure migration, but simply looks at very fast fluctuations in the adhesion state of the cell, i.e. the cell is relatively fixed in one place, but the bias for close focal adhesions changes from one cell edge to the opposite. These types of adhesion changes can occur on a much faster time scale than actual migration, and may act as a substitute for having to actually observe cell movement over a specific distance, and hence may add advantages to the assay in this format.

There are various parameters by which movement of cells in response to oscillating gradients can be described.

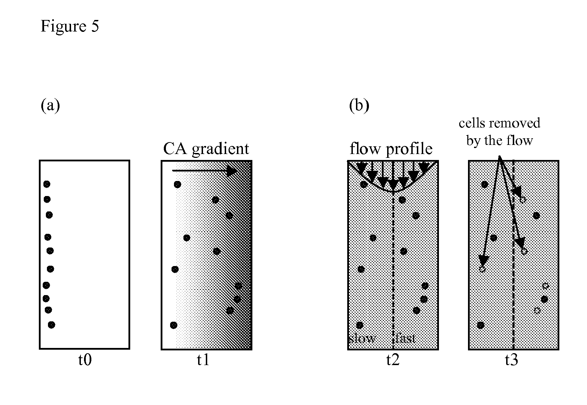

One such parameter is cell trajectories. In order to analyze the trajectories of a cell, the motion of each cell is observed and only the component along the axis of the chemotactic agent gradient which changes direction with the same period as the oscillating gradient is extracted as chemotactic movement. This is schematically depicted in Fig. 5 a) where it is shown that a cell changes its migratory behavior as a response to a reverse direction of the gradient. If this is not the case, the movement is not classified as chemotactic, but as chemokinetic movement.

Cell trajectories for describing chemotactic behavior are a valuable parameter. As has already mentioned above, chemotactic mobility of certain cell types such as monocytes seems to be different for cells that are derived from patients suffering by coronary artery diseases such as atherosclerosis or healthy individuals. Thus, by measuring trajectories of blood cells such as monocytes, macrophages and neutrophils derived from patients suffering from diseases such as chronic artery diseases and cancer versus healthy individuals, it is possible to develop profiles that are indicative of the disease state based on the chemotactic migratory behavior of the cells and can thus be used for diagnosis. In principle, one can also use such a method for any adherence cell line as well (tissue and tumor biopsy samples, endothelial, epithelial, fibroblast) and measure the adherence tendency by looking at focal spot adhesion dynamics as mentioned above.

It is to be understood that in the context of the present invention the use of such parameters for diagnostic purposes refers to diseases for which it is known that the disease mechanism at least partially involves chemotactic migratory behavior of cells. Such diseases are the aforementioned coronary artery diseases including arteriolosclerosis and atherosclerosis as well as certain type of cancers such as colorectal, lung, prostate and breast cancer. Other cancers may include leukemia, lymphoma, head and neck cancer, non-small cell lung cancer, ovarian cancer, urinary bladder cancer, kidney cancer, cervical cancer etc.

Particularly for coronary artery diseases such as atherosclerosis and certain types of cancer, it is known that blood cells such as monocytes and macrophages play a major contributory role during disease development. For example in atherosclerosis, monocytes are recruited by a host of cellular factors such as VEGF and VCAM-1 and then invade into the epithelium. By differentiation and cholesterol uptake mechanisms, the monocytes ultimately turn into foam cells, which may contribute to plaque rupture. Similarly, in certain types of cancer, tumour formation is characterised by an aberrant increase of angiogenic activity that is necessary in order to ensure that the tumour receives sufficient blood supply. Angiogenesis, however, is known to rely at least partially on recruitment of cells such blood cells including particularly monocytes and neutrophils.

Therefore, the present method of detecting movement of cells can be used to determine parameters such as the trajectories of monocytes derived of patients suffering from a disease such as coronary artery diseases versus healthy individuals and to use differences in the trajectories to make a diagnosis as to the occurrence and the state of such a disease.

Another parameter which may be determined by monitoring movement of cells with response to oscillating gradient is the time lag between switching the chemotactic agent gradient and a response of individual cells. This is schematically depicted in Fig. 5b). The time lag is also considered to be a biomarker for diseases such as the above-mentioned diseases that are characterized in that certain types of cells differ with respect to their migratory capacity if derived from patients versus healthy individuals. In particular, the time lag parameter may be used to analyze blood cells such as monocytes and neutrophils of patients suffering from coronary artery diseases such as atherosclerosis versus healthy individuals. The data obtained in such comparisons may be used in the diagnosis of the specific disease.

Yet another parameter that can be used to reflect migratory response to oscillating gradient chemotactic agents is the adhesion tendency. As monocyte adhesion has been shown to be different in patients for different degrees of coronary collateral circulations, adhesion tendency is also an interesting parameter in the context of diagnosing such diseases.

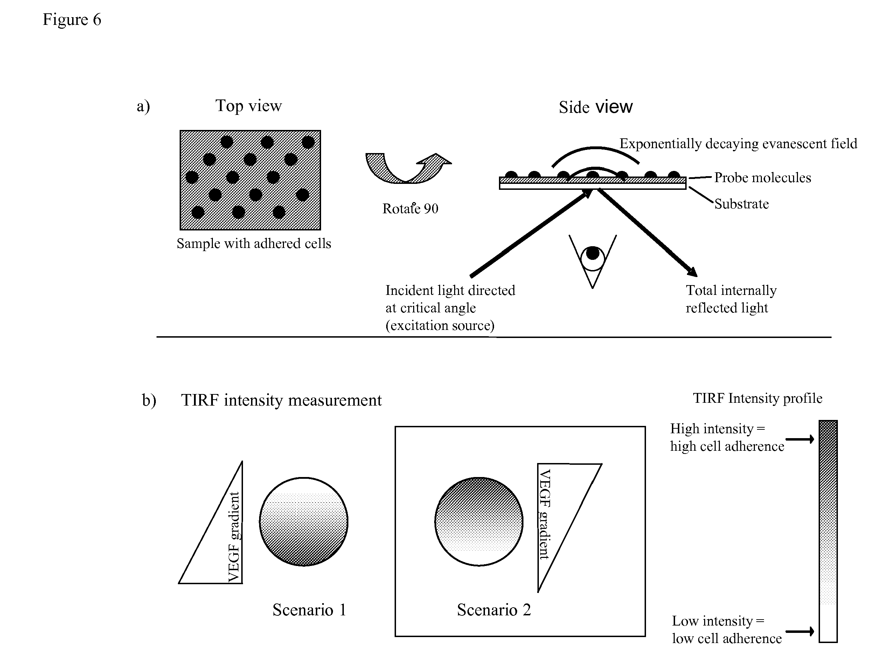

To measure cell adhesion, one would first load e.g. a device as set out above and establish a gradient and monitor movement of the cells in response to the gradient. One would then e.g. establish a lateral flow in order to change the direction of the gradient and to determine cells, which do not adhere but move with the flow. This approach is schematically depicted in Fig. 6.

Moreover, this approach allows assessing the relationship between adhesion tendency and cell mobility. Thus, those cells which will be able to respond to the initial gradient in a certain time period may be considered to be fast cells. If then the direction of the gradient is altered by e.g. applying a lateral flow, which in the specific embodiment may result in a destruction of the gradient, the number of cells that adhere can be determined. Thus it will be possible to determine the relative portion of cells, which have a high migratory capacity, i.e. react fast to the gradient and adhere versus those cells, which have a slow migratory capacity and adhere. The same can be, of course, done to identify cells with high and slow migratory capacity, which do not adhere.

As these parameters are also likely to differ for those cell types such as blood cells including particularly monocytes that are involved in the above-mentioned types of diseases, the parameters deduced can also be used in disease diagnosis.

Thus, the present invention is directed to a method of monitoring the movement of cells in response to chemotactic agents with the method being characterized in that a first gradient is established and movement of cells with respect to that gradient is monitored and that then the direction and/or the extent of the gradient is changed with movement of cells in response to that second gradient also being monitored. In a preferred embodiment, one uses gradients of reverse directions, which constantly change over time. These oscillating gradients are particularly useful to analyze the migratory capacity of cells.

The parameters that are retrieved using these approaches can then be used e.g. for diagnostic purposes.

Thus, the present invention in one embodiment relates to a method of diagnosing and/or staging a disease comprising at least the steps of:
a) Providing at least one support structure;
b) Providing a first sample with at least one cell which is derived from a human or animal subject suspected of suffering from the disease in question;
c) Allowing said at least one cell of said first sample to reversibly adhere to at least a part of said support structure
d) Establishing a gradient of a chemotactic agent, which is capable of inducing chemotaxis of said at least one cell of said first sample along of at least a part of said support structure;
e) Monitoring movement of said at least one cell of said first sample to or from said gradient;
f) Reversing the direction of said gradient;
g) Monitoring movement of said at least one cell of said first sample to or from said gradient;
h) Deciding on the disease state of the human or animal being on the findings obtained in steps a) to g).

Deciding on the occurrence and/or the grade of the disease in step h) may be undertaken by different approaches. Thus, one may repeat steps a) to g) for a patient at different times to establish a pattern of movement that is characteristic for the patient's cells. If one observes a change in the movement behaviour over time this may be an indication of ongoing disease development.

In addition or alternatively one may compare the data obtained in steps a) to g) with a suitable control of healthy subjects. In this embodiment the invention relates to a method of diagnosing a disease comprising at least the steps of:
a) Providing at least one support structure;
b) Providing a first sample with at least one cell which is derived from a human or animal subject suspected of suffering from the disease in question;
c) Allowing said at least one cell of said first sample to reversibly adhere to at least a part of said support structure
d) Establishing a gradient of a chemotactic agent, which is capable of inducing chemotaxis of said at least one cell of said first sample along of at least a part of said support structure;
e) Monitoring movement of said at least one cell of said first sample to or from said gradient;
f) Reversing the direction of said gradient;
g) Monitoring movement of said at least one cell of said first sample to or from said gradient;
h) Providing a second sample with at least one cell which is derived from a human or animal subject not suffering from the disease in question;
i) Allowing said at least one cell of said second sample to reversibly adhere to at least a part of said support structure
j) Establishing a gradient of a chemotactic agent, which is capable of inducing chemotaxis of said at least one cell of said second sample along of at least a part of said support structure;
k) Monitoring movement of said at least one cell of said second sample to or from said gradient;
l) Reversing the direction of said gradient;
m) Monitoring movement of said at least one cell of said second sample to or from said gradient;
n) Determining the difference in the movement of said at least one cell of said first and said second sample;
o) Deciding on the disease state of the human or animal being from which said first sample has been derived.

In a preferred embodiment all these of the aforementioned steps are carried out outside the human or animal body.

In another embodiment the present invention relates to a method of data acquisition comprising at least the steps of:
a) Providing at least one support structure;
b) Providing a first sample with at least one cell which is derived from a human or animal subject suspected of suffering from the disease in question;
c) Allowing said at least one cell of said first sample to reversibly adhere to at least a part of said support structure
d) Establishing a gradient of a chemotactic agent, which is capable of inducing chemotaxis of said at least one cell of said first sample along of at least a part of said support structure;
e) Monitoring movement of said at least one cell of said first sample to or from said gradient;
f) Reversing the direction of said gradient;
g) Monitoring movement of said at least one cell of said first sample to or from said gradient.

One may use the method of data acquisition again to either monitor a subject over a period of time by repeating steps a) to g) and/or to compare the data with those obtained for a control. In this latter case, the invention relates to a method of data acquisition comprising at least the steps of:
a) Providing at least one support structure;
b) Providing a first sample with at least one cell which is derived from a human or animal subject suspected of suffering from the disease in question;
c) Allowing said at least one cell of said first sample to reversibly adhere to at least a part of said support structure
d) Establishing a gradient of a chemotactic agent, which is capable of inducing chemotaxis of said at least one cell of said first sample along of at least a part of said support structure;
e) Monitoring movement of said at least one cell of said first sample to or from said gradient;
f) Reversing the direction of said gradient;
g) Monitoring movement of said at least one cell of said first sample to or from said gradient;
h) Providing a second sample with at least one cell which is derived from a human or animal subject not suffering from the disease in question;
i) Allowing said at least one cell of said second sample to reversibly adhere to at least a part of said support structure
j) Establishing a gradient of a chemotactic agent, which is capable of inducing chemotaxis of said at least one cell of said second sample along of at least a part of said support structure;
k) Monitoring movement of said at least one cell of said second sample to or from said gradient;
l) Reversing the direction of said gradient;
m) Monitoring movement of said at least one cell of said second sample to or from said gradient;
n) Determining the difference in the movement of said at least one cell of said first and said second sample.

In one embodiment of the present invention, all of the above-mentioned steps may be performed outside the human or animal body.

It has been set out above that the present invention relies on determining the response of cells to gradients of chemotactic agents that change their direction and/or extent over time. The migratory response of cells to oscillating gradient constitutes a preferred embodiment of the present invention.

Of course, as has been set out above, the response, i.e. the movement of cells towards or away from the chemotactic agents has to be monitored. The person skilled in the art is aware of various monitoring techniques including e.g. light microscopy.

However, the inventors of the present invention have further found that two approaches are particularly suited to exactly monitor the migratory behavior of a cell in response to a chemotactic agent.

The present invention in one embodiment therefore relates to the detection of the movement of a cell in response to a chemotactic agent by Total Internal Reflection Microscopy (TIRFM).

TIRFM employs the properties of an induced evanescent wave to selectively illuminate a fluorophore in a restricted specimen region immediately adjacent to a glass-fluid, particularly a glass-water or glass-buffer interface. The basis concept of TIRFM requires only an excitation light beam traveling at high incident angle through the support structure on which the cells adhere as has been set out above.

Refractive index differences between the glass and water phases regulate how light is refracted or reflected at the interface as a function of incident angle. At a specific critical angle, the beam of light is totally reflected from the glass/fluid interface, rather than passing through and refracting in accordance with Snell's law. This total reflection generates a very thin electromagnetic field that is usually less than 200 nm in dimension in the fluid medium, which has an identical frequency to that of the incident light.

This field that is also called the evanescent wave or field undergoes exponential intensity decay with increasing distance from the surface. The inventors of the present invention have found that labeling the cells for which movement is to be examined with a detectable marker may allow to spectroscopically detecting the evanescent wave generated by TIRFM.

In a preferred embodiment the cells are thus labeled with fluorescent dyes. To this end one may either label cellular structures such as cell surface receptors with a fluorescent dye or one may use fluorophores that are known to associate with cells. Typical fluorescent markers can include Cy5, Cy3, Texas Red, FITC, Attodye, Cydye, Alexa647, Alexa 488, Alexa 546, Alexa 546, Alexa 594, Alexa 633, GFP, YFP, CFP and dsRED. A particularly suitable fluorophore is the life-cell-tracker from Invitrogen. Other markers may be Q-dots or nanoparticles

If such a labeled cell is brought into contact with the above-mentioned support structure and a gradient is established the cells will start to crawl or roll along the surface of the device. Adherence of the cell may be mediated as mentioned above by probe molecules. If cells which carry at a detectable fluorescent marker and which have adhered to the support structure are analyzed using TIRFM one will observe an evanescent field which is amplified if an evanescent field created by TIRFM stimulates the fluorescent marker. If then the direction of the chemotactic stimulus is changed as mentioned above, the cell will start to crawl in a different direction. This will lead to a reduced interaction between the evanescent field of TIRFM and the fluorescent marker resulting in a reduced detected intensity.

At the same time the leading edge of the cell, which mediates crawling, may get into closer contact and the evanescent field may now then stimulate the fluorophore at a different location of the crawling cell resulting in an increased intensity.

Thus, by first establishing e.g. the gradient with an increasing concentration of a chemoattractant agent such as VEGF, the leading edge of a cell such as a monocyte will make close adhesion contact with the surface of the sample while the lagging edge of the cell will unadhere in order to facilitate movement of the cell towards the higher concentration of the gradient. Given that TIRFM is sensitive to the distance between the cell and the surface, very slight changes in cell adhesion can be resolved by fluorescent intensity at the focal adhesion between the cell and the support surface. By using oscillating gradients, which induce the cells to constantly move around on the support structure it is thus possible to very accurately determine the migratory behavior of cells using TIRFM. This approach is schematically depicted in Fig. 6.

Another approach which may be used to very accurately monitor the response in the cells migratory behavior in response to a chemotactic stimulus is Förster Resonance Energy Transfer (FRET). FRET is based on the principle that a donor fluorophore and an acceptor fluorophore are brought into close proximity. Then the donor fluorophore is excitated at the donor fluorophore's excitation wavelength and an energy transfer between the donor fluorophore and the acceptor fluorophore occurs if both fluorophores are in sufficient close proximity and orientation. The energy transfer can be observed at the acceptor fluorophore's emission wavelength, which is different from the donor fluorophore's excitation wavelength.

In one embodiment of the present invention that uses FRET for detecting cell movement one may thus label the support structure with e.g. the donor fluorophore and the cells for which movement is to be analyzed with the acceptor fluorophore. Of course, the direction of the labeling may be reversed so that the support structure comprises the acceptor fluorophore while the cell is labeled with the donor fluorophore.

The person skilled in the art is also aware that it is not mandatory to label the surface of the support structure but that labeling probe molecules that have been immobilized on the support structure may also be used for labeling with the donor or the acceptor fluorophore.

As has been discussed above for TIRFM, one then allows cells to adhere to the surface of the support structure and establishes e.g. a gradient of a chemoattractant agent. The cells, which have adhered to the support structure, will start to move the direction of increasing concentrations of the chemoattracting agents. Thus, the leading edge of the cells moving towards the increasing concentration will make close contact to the surface of the support structure while the lagging edge will dispatch from the support structure. That in turn means that the fluorophores that are used for labeling the cells and which may be the same type of fluorophores that has been mentioned above for TIRFM will make close contact with the fluorophores being disposed on the surface of the structure in case of the leading edge and will not be in close proximity for the lagging edge. If e.g. a cell is labeled with an acceptor fluorophore and the support structure is labeled with a donor fluorophore one will thus be able to observe the leading edge of the crawling cells by FRET if one uses a donor excitation wavelength because for the leading edge, an energy transfer from the donor fluorophore to the acceptor fluorophore will occur so that emission at the acceptor fluorophore wavelength can be observed. At the same time, the lagging edge will not be in close proximity with the consequence that no FRET signal is observed or at least a signal of significantly reduced intensity is observed. The donor and acceptor pairs are, of course, interchangeable, i.e. the donor may be on a cell and an acceptor may on the substrate surface or vice versa.

The person skilled in the art is capable of selecting appropriate donor and acceptor fluorophore and may rely in this context inter alia on the fluorescent markers that have been mentioned above in the context of TIRFM. Further, the person skilled in the art is familiar with the coupling chemistry that may be used to adhere the fluorophores to the support structure, the probe molecules thereon and the cells. In principle, covalent linking of fluorophores to these different entities may be undertaken as described above for coupling probe molecules to the support structure. Appropriate FRET pairs can be Alexa 488 and 546, or Alexa 546 and 594.

The present invention provides for numerous advantages. It has been recognized that the migratory behavior of certain cell types differs for patients suffering from diseases such as coronary artery diseases versus healthy persons. However, the test systems, which have been used so far sample, do not allow for a clear distinction between chemotactic versus chemokinetic movement. At least this differentiation is rather complicated and time-consuming. By using the methods in accordance with the present invention which rely on gradients of chemotactic agents which differ with respect to the orientation and/or extent over time and which preferably are oscillating gradients of reverse direction, it is possible to study valuable parameters of cell migratory behavior in a lot more efficient and convenient way. Further, these parameters can be reliably determined and may be used to develop diagnostic markers that can be helpful in the diagnosis of coronary artery diseases including atherosclerosis. As mentioned above, one can use the present invention to measure fluctuations in adhesion state which is a fast approach to characterize the migratory behavior of a cell.
While the above invention has been described with respect to some of its preferred embodiments, this is in no way to limit the scope of the invention. The person skilled in the art is clearly aware of further embodiments and alterations to the previously described embodiments that are still within the scope of the present invention.

## Claims

1. Method of detecting movement of at least one cell comprising at least the steps of:
a) Providing at least one support structure;
b) Providing a sample with at least one cell;
c) Allowing said at least one cell to reversibly adhere to at least a part of said support structure
d) Establishing a gradient of a chemotactic agent, which is capable of inducing chemotaxis of said at least one cell along of at least a part of said support structure;
e) Monitoring movement and/or adherence of said at least one cell to or from said gradient;
f) Changing the direction of said gradient;
g) Monitoring movement of said at least one cell to or from said gradient.

2. Method according to claim 1,
wherein the direction of the gradient is reversed in step e).

3. Method according to claim 1 or 2,
wherein steps d) to g) are repeated over time to monitor movement of said at least one cell in response to an oscillating gradient.

4. Method according to any of claims 1 to 3,
wherein the support structure comprises at least one probe molecule that allows said at least one cell to adhere to said support structure.

5. Method according to claim 4,
wherein said at least one probe molecule is selected from the group comprising small molecules, peptides, glycopeptides, proteins, glycoproteins, receptors, receptor ligands, antibodies, growth factors, hormones, cytokines, chemokines, cell adhesion molecules, integrins, vitamins, nucleic acids and derivatives thereof, lipids and glycans.

6. Method according to claim 4 or 5,
wherein said at least one probe molecule and said at least one chemotactic agent are identical.

7. Method according to any of claims 1 to 6,
wherein monitoring movement of said at least one cell includes determination of the trajectories, the time lag of responding to a change in the gradient, the adhesion tendency and/or the mobility of said at least one cell.

8. Method according to any of claims 1 to 7,
wherein movement of said at least one cell is measured using Total Internal Reflection Microscopy (TIRFM).

9. Method according to claim 8,
wherein said at least one cell is labeled with at least one detectable marker.

10. Method according to claim 9,
wherein said at least one detectable marker is a fluorescent marker.

11. Method according to any of claims 1 to 7,
wherein movement of said at least one cell is measured using Förster Resonance Energy Transfer (FRET).

12. Method according to claim 11,
wherein said at least one cell and said at least one support structure or wherein said at least one cell and said at least one probe molecule are labeled with different fluorescent markers that are suitable to function as donor and acceptor fluorophores:

13. An apparatus comprising:
- a first inlet and a second inlet from which at least first generation of channels originates wherein said first generation comprises at least two first generation channels;
- a first common channel providing communication between each of the at least two first generation channels; and
- at least a second generation of channels comprising at least three second generation channels, each second generation channel having a first end and a second end, the first end of each being in communication with the first common channel and the second end of each being in communication with a second common channel,
- a third inlet; and
- an outlet;
wherein the apparatus is adapted to allow formation of a liquid gradient of a component by connecting the first inlet at least to one fluid comprising a component at a first concentration and by connecting the second inlet at least to one fluid comprising said component at a second concentration wherein the first and second concentration of said component differ; and
wherein said first inlet and said second inlet are adapted to allow for reversal of the orientation of the gradient.

14. Apparatus according to claim 13,
wherein said first and second inlet are three way valves.

15. Use of an apparatus according to claim 13 or 14 for detecting cell movement.
